# EUROPEAN PATENT APPLICATION

(11) **EP 4 371 491 A1**
(43) Date of publication of application: **22.05.2024**
(21) Application number: 22858758.0
(22) Date of filing: 17.08.2022
(51) Int. Cl.: A61B 5/346, A61B 5/327, A61B 5/28, A61B 5/00, G16H 50/50, G16H 50/20, G16H 50/70, G06N 20/00

(54) **ELECTROCARDIOGRAM READING SYSTEM IN WHICH DEEP LEARNING-BASED MODEL AND RULE-BASED MODEL ARE INTEGRATED**

(30) Priority: 17.08.2021 KR 20210107777
(71) Applicant: Medicalai Co., Ltd., Seoul 06302 (KR)
(72) Inventor: KWON, Joon Myoung, Seoul Seoul 06629 (KR)
(74) Representative: Isarpatent
(86) International application number: PCT/KR2022/012300
(87) International publication number: WO 2023/022521

(57) **Abstract**

The present invention discloses an electrocardiogram reading system in which a deep learning-based model and a rule-based model are integrated, the electrocardiogram reading system including: an electrocardiogram measurement unit (110) configured to generate electrocardiogram data by measuring 1- or more-lead electrocardiograms; a deep learning-based prediction unit (120) configured to generate disease prediction information by diagnosing and predicting disease from the electrocardiogram data through a deep learning algorithm; a rule-based inference unit (130) configured to generate disease inference information by inferring disease from the electrocardiogram data input from the electrocardiogram measurement unit (110) through a rule-based algorithm; and an integrated reading unit (140) configured to generate reading information by performing disease reading through the integrated analysis of the disease prediction information and the disease inference information and provide diagnostic information descriptive of the reason and basis for the diagnosis of the disease.

## Description

### Technical Field

The present invention relates to an electrocardiogram reading system in which a deep learning-based model and a rule-based model are integrated, which prevents the misreading of electrocardiograms and enables rapid and accurate reading.

### Background Art

Recently, due to the development of big data and artificial intelligence technology, artificial intelligence has been actively used in the medical and healthcare fields. In particular, the development of deep learning technology has made it possible to utilize unstructured data such as sounds, pictures, signals, and videos.

Meanwhile, unlike conventional classical machine learning methods, feature learning is enabled. Accordingly, accuracy can be improved by extracting various features from data without human prejudice. While classical machine learning requires significant amounts of labor and time from experts to extract features, deep learning makes it possible to develop algorithms rapidly with less effort.

However, deep learning has a limitation in that the reason for determination cannot be known due to the black box problem, which may lead to a medical accident. Therefore, there is a demand for technology that prevents the misreading of electrocardiograms and enables rapid and accurate reading.

### Disclosure

### Technical Problem

A technical problem to be overcome by the spirit of the present invention is to provide an electrocardiogram reading system in which a deep learning-based model and a rule-based model are integrated, in which the misreading of electrocardiograms is prevented from being performed by a deep learning-based prediction unit by setting a readable range for final reading by a rule-based inference unit, and the possibility of misreading and the accuracy of diagnosis are complemented by each other by enabling rapid and accurate reading to be performed by the deep learning-based prediction unit.

### Technical Solution

In order to overcome the above technical problem, an embodiment of the present invention provides an electrocardiogram reading system in which a deep learning-based model and a rule-based model are integrated, the electrocardiogram reading system including: an electrocardiogram measurement unit configured to generate electrocardiogram data by measuring 1- or more-lead electrocardiograms; a deep learning-based prediction unit configured to generate disease prediction information by diagnosing and predicting disease from the electrocardiogram data input from the electrocardiogram measurement unit through a deep learning algorithm constructed by being trained on training datasets of 1- or more-lead electrocardiograms and diseases corresponding to these electrocardiograms; a rule-based inference unit configured to generate disease inference information by inferring disease from the electrocardiogram data input from the electrocardiogram measurement unit through a rule-based algorithm including a knowledge base, constructed with electrocardiogram data and disease information corresponding to the electrocardiogram data, and inference rules; and an integrated reading unit configured to generate reading information by performing disease reading through the integrated analysis of the disease prediction information and the disease inference information and provide diagnostic information descriptive of the reason and basis for the diagnosis of the disease.

In this case, the rule-based inference unit may set a readable range for reading performed by the integrated reading unit.

Furthermore, when the disease prediction information generated by the deep learning-based prediction unit is included in the readable range, the integrated reading unit may generate and output the reading information and the diagnostic information; when the disease prediction information generated by the deep learning-based prediction unit is not included in the readable range, electrocardiograms may be re-measured by the electrocardiogram measurement unit, or the disease prediction information may be re-generated by readjusting parameters of the deep learning-based prediction unit.

Furthermore, the integrated reading unit may perform final reading by using only the disease inference information generated by the rule-based inference unit.

Furthermore, the final diagnosis of the corresponding disease for the electrocardiogram data may be made by the deep learning-based prediction unit and the rule-based inference unit.

Primary reading information may be generated by provisionally reading the electrocardiogram data through the deep learning-based prediction unit or the rule-based inference unit; and the integrated reading unit may include: a first reading unit configured to receive the primary reading information, and to allow a nurse, clinical pathologist, or emergency technician to log in, read the primary reading information, and generate secondary reading information; and a second reading unit configured to receive the secondary reading information, and to allow a cardiologist to log in, read the secondary reading information and generate final reading information.

### Advantageous Effects

According to the present invention, there are effects in that the misreading of electrocardiograms is prevented from being performed by the deep learning-based prediction unit by setting the readable range for final reading performed by the rule-based inference unit, the possibility of misreading and the accuracy of diagnosis are complemented by each other by enabling rapid and accurate reading to be performed by the deep learning-based prediction unit, the possibility of medical accidents is reduced by providing diagnostic information descriptive of the basis and reason for the diagnosis of disease, rapid medical response is enabled by rapidly performing final reading based on previous reading, and various healthcare services are enabled by applying the present invention not only to the medical field but also to the healthcare field utilizing various types of biometric data.

### Description of Drawings

FIG. 1 illustrates a block diagram of an electrocardiogram reading system in which a deep learning-based model and a rule-based model are integrated according to an embodiment of the present invention;
FIG. 2 illustrates an implementation diagram of the electrocardiogram reading system in which a deep learning-based model and a rule-based model are integrated, which is shown in FIG. 1; and
FIG. 3 illustrates the configuration of the integrated reading unit of the electrocardiogram reading system in which a deep learning-based model and a rule-based model are integrated, which is shown in FIG. 1.

### Mode for Invention

Embodiments of the present invention having the above-described features will be described in more detail below with reference to the accompanying drawings.

An electrocardiogram reading system in which a deep learning-based model and a rule-based model are integrated according to an embodiment of the present invention includes: an electrocardiogram measurement unit 110 configured to generate electrocardiogram data by measuring 1- or more-lead electrocardiograms; a deep learning-based prediction unit 120 configured to generate disease prediction information by diagnosing and predicting disease from the electrocardiogram data input from the electrocardiogram measurement unit 110 through a deep learning algorithm constructed by being trained on training datasets of 1- or more-lead electrocardiograms and diseases corresponding to these electrocardiograms; a rule-based inference unit 130 configured to generate disease inference information by inferring disease from the electrocardiogram data input from the electrocardiogram measurement unit 110 through a rule-based algorithm including a knowledge base, constructed with electrocardiogram data and disease information corresponding to the electrocardiogram data, and inference rules; and an integrated reading unit 140 configured to generate reading information by performing disease reading through the integrated analysis of the disease prediction information and the disease inference information and to provide diagnostic information descriptive of the reason and basis for the diagnosis of the disease. The gist of the present invention is to prevent the misreading of electrocardiograms and enable rapid and accurate reading.

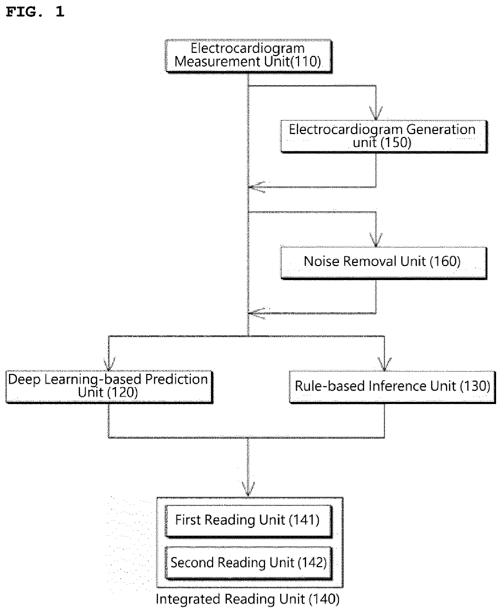

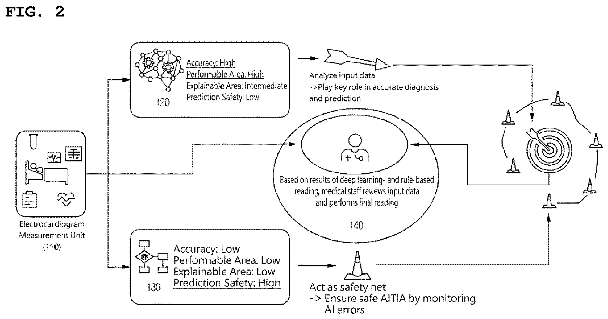

The electrocardiogram reading system in which a deep learning-based model and a rule-based model are integrated, which is configured as described above, will be described in detail below with reference to FIGS. 1 to 3 as follows.

First, the electrocardiogram measurement unit 110 is a component configured to generate electrocardiogram data by measuring 1- or more-lead electrocardiograms. The electrocardiogram measurement unit 110 is provided with two electrodes, acquires 1- or more-lead electrocardiogram data corresponding to one or more electrical axes in such a manner as to bring the two electrodes into contact with two portions of the body of an examinee, and then transmits the acquired 1- or more-lead electrocardiogram data to the deep learning-based prediction unit 120 and the rule-based inference unit 130 through wired/wireless short-distance communication, thereby enabling electrocardiograms to be measured even in daily life.

For example, a plurality of electrocardiograms for at least two different electrical axes may be measured by measuring a lead-I electrocardiogram corresponding to one electrical axis in such a manner as to bring the respective electrodes into contact with both hands and also measuring a lead-II electrocardiogram corresponding to the other electrical axis in such a manner as to bring the respective electrodes into contact with the right and left ankles, which are different from the previous body combination for contact with the electrodes.

Furthermore, the electrocardiogram measurement unit 110 may include a wearable electrocardiogram patch 111, a smartwatch 112 or a 6-lead electrocardiogram bar for short-term measurement capable of contact or contactless electrocardiogram measurement during daily life, or an electrocardiogram measurement device installed in a medical institution and configured to measure 1- or more-lead electrocardiograms, and may thus measure synchronous or asynchronous electrocardiograms.

In this case, the electrocardiogram measurement unit 110 may measure successive electrocardiograms of an examinee and transmit them to an electrocardiogram generation unit 150, or may measure two electrocardiograms at different times and transmit them to the deep learning-based prediction unit 120 and the rule-based inference unit 130.

Next, through a deep learning algorithm previously constructed by being trained on training datasets of 1- or more-lead electrocardiograms and diseases corresponding to the 1- or more-lead electrocardiograms, which are big data accumulated in servers of medical institutions, the deep learning-based prediction unit 120 generates disease prediction information by diagnosing and predicting a corresponding disease from the electrocardiogram data input from the electrocardiogram measurement unit 110.

For example, various deep learning algorithms such as CNN, LSTM, RNN, and MLP may be applied to the deep learning-based prediction unit 120. The deep learning-based prediction unit 120 may generate disease prediction information with the risk level of a disease classified as a specific stage.

Next, the rule-based inference unit 130 is an expert system that makes inferences using the big data collected and organized in the servers of the medical institutions. The rule-based inference unit 130 infers a disease from the electrocardiogram data input from the electrocardiogram measurement unit 110 and then generates disease inference information through a knowledge base constructed with electrocardiogram data and corresponding disease information and a rule-based algorithm composed of inference rules such as inductive inference, deductive inference, and analogical inference.

In this case, the rule-based inference unit 130 may generate disease inference information with the risk level of the disease classified as a specific stage.

Meanwhile, the rule-based inference unit 130 may generate a boundary out of which misreading can be caused from the electrocardiogram data by setting a readable range for the final reading performed by the integrated reading unit 140.

Next, the integrated reading unit 140 generates reading information by performing the final reading of the disease through the integrated analysis of the disease prediction information and the disease inference information, and provides diagnostic information descriptive of the basis and reason for the diagnosis of the disease.

For example, the primary reading information of the disease prediction information and the disease inference information is generated by provisionally reading the electrocardiogram data through the deep learning-based prediction unit 120 or the rule-based inference unit 130. Next, the integrated reading unit 140 may include a first reading unit 141 configured to receive the primary reading information and to allow a nurse, clinical pathologist, or emergency technician to log in, read the primary reading information, and generate secondary reading information, and a second reading unit 142 configured to receive the secondary reading information and to allow a cardiologist to log in, read the secondary reading information, and finally generate reading information.

Meanwhile, when the disease prediction information generated by the deep learning-based prediction unit 120 is within the readable range previously set by the rule-based inference unit 130, the integrated reading unit 140 may generate and output reading information and diagnostic information. When the disease prediction information generated by the deep learning-based prediction unit 120 is not within the readable range previously set by the rule-based inference unit 130, electrocardiograms may be re-measured by the electrocardiogram measurement unit 110, or the disease prediction information may be re-generated by readjusting the parameters of the deep learning algorithm of the deep learning-based prediction unit 120.

Furthermore, the integrated reading unit 140 may perform final reading based on only the disease inference information, generated by the rule-based inference unit 130, excluding the disease prediction information.

Alternatively, the disease may be finally diagnosed based on the electrocardiogram data only by the deep learning-based prediction unit 120 and the rule-based inference unit 130 without the final reading performed by the integrated reading unit 140.

Meanwhile, the integrated reading unit 140 may receive the disease prediction information and disease inference information generated with the risk levels of diseases divided into stages, and may finally determine the risk level of the disease. Accordingly, the integrated reading unit 140 may divide the risk levels of diseases into three stages: a normal stage, which does not require additional reading performed by a nurse or medical specialist; an abnormal stage, which requires additional reading performed by a nurse or medical specialist; and a critical stage, which requires urgent analysis performed by a medical specialist, and may classify and determine the risk level of the disease according to treatment priority.

In this case, the final risk level of the disease may be determined by collectively and arithmetically averaging the risk level quantified by the deep learning-based prediction unit 120 and the risk level quantified by the rule-based inference unit 130, or may be determined by assigning a weight to any one risk level and then arithmetically averaging all risk levels.

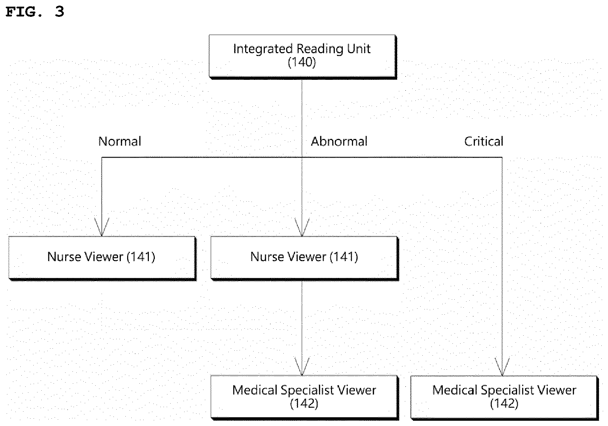

For example, referring to FIG. 3, when the final determination result of the risk level is a normal stage, the integrated reading unit 140 does not read the electrocardiogram data or transmits the electrocardiogram data only to the nurse viewer 143 so that reading can be performed and completed by a nurse. In the case of an abnormal stage, sequentially, the electrocardiogram data is transmitted to the nurse viewer 143 so that first reading is performed by a nurse, and then the electrocardiogram data is transmitted to the medical specialist viewer 144 so that second reading can be performed and completed by a medical specialist. In the case of a critical stage, the electrocardiogram data, together with emergency reading alarm information, may be immediately transmitted to the medical specialist viewer 144 so that reading can be performed rapidly.

Furthermore, when the final determination result is an abnormal or critical stage, the integrated reading unit 140 transmits the electrocardiogram data to the nurse viewer 143 or medical specialist viewer 144 so that each waveform corresponding to the abnormal or critical stage is displayed to be specially marked or overlap reference normal range electrocardiogram data in the electrocardiogram data. Accordingly, a nurse or medical specialist is allowed to intensively analyze the waveform and rapidly and intuitively identify the features that serve as the basis for the determination.

Furthermore, when the final determination result of the electrocardiogram data is a normal stage but abnormal findings are suspected upon reading performed by the nurse viewer 143, the risk level is upgraded to an abnormal or critical stage, and the electrocardiogram data is transmitted to the medical specialist viewer 144 so that rapid reading can be performed by a medical specialist in response to emergency reading alarm information.

Meanwhile, there may be further included the electrocardiogram generation unit 150 configured to generate residual electrocardiogram data from the 1- or more-lead electrocardiogram data provided by the electrocardiogram measurement unit 110. Accordingly, the accuracy of the prediction performed by the deep learning-based prediction unit 120 and the accuracy of the inference performed by the rule-based inference unit 130 may be further improved.

In this case, the electrocardiogram generation unit 150 may generate synchronous or asynchronous multiple-lead electrocardiogram data by identifying the feature information of 1- or more-lead synchronous or asynchronous electrocardiogram data and synthesizing a plurality of pieces of residual lead electrocardiogram data.

Furthermore, there may be further included a noise removal unit 160 in order to further increase the reliability of the disease information generated by the electrocardiogram generation unit 150 by minimizing noise in the electrocardiogram data provided from the electrocardiogram measurement unit 110 to the electrocardiogram generation unit 150. For example, through a deep learning algorithm constructed by being pre-trained on datasets of electrocardiogram data for individual leads having less noise and the unique styles of the electrocardiogram data for individual leads based on a large amount of electrocardiogram data, such as the standard 12-lead electrocardiogram data accumulated in medical institutions, the noise removal unit 160 may extract a corresponding unique style from the electrocardiogram data measured by the electrocardiogram measurement unit 110 with the characteristics of an examinee and the characteristics of a measurement method reflected therein, and may perform conversion into and generate electrocardiogram data of a specific lead style containing no noise through the extracted unique style.

Furthermore, the noise removal unit 160 may identify the unique style of each electrocardiogram data for each lead with high accuracy with the characteristics of the examinee attributable to age, gender, disease, etc. and the characteristics of the measurement method attributable to the attachment locations of electrodes, an electrocardiogram device, etc. reflected therein, and may perform conversion into and generate electrocardiogram data for each lead based on the identified unique style more accurately.

Furthermore, the deep learning-based prediction unit 120 and the rule-based inference unit 130 may receive synchronous or asynchronous electrocardiogram data and generate disease prediction information and disease inference information, respectively, or may generate the electrocardiogram data represented in the form of a graph or the electrocardiogram data quantified through a specific formula and generate disease prediction information and disease inference information, respectively.

Furthermore, the deep learning-based prediction unit 120 and the rule-based inference unit 130 may integrate two or more pieces of electrocardiogram data into one piece of electrocardiogram data and then input the one piece of electrocardiogram data, or input two or more electrocardiograms into respective algorithms, may extract measured semantic features, that is, spatial and time-series features, midway, and may compare two electrocardiograms based on the measured semantic features, thereby measuring, diagnosing, examining, and predicting a health condition at the time when the measurement was made by the electrocardiogram measurement unit 110 or a future health condition.

In this case, a method of integrating electrocardiograms measured at two or more times is to divide a single electrocardiogram for respective beats and then pair and input beats for the same lead measured at different times, or to compare semantic features or result values extracted after the input.

Alternatively, it may be possible to combine pieces of electrocardiogram data themselves without dividing the electrocardiograms measured at two or more times into beats. The pieces of electrocardiogram data measured at two or more times may be combined and then input. The electrocardiogram data may be divided for respective leads, combined together, and then input. The electrocardiograms measured at different times may be input into respective algorithms, semantic features or output values may be extracted and combined, and then a final conclusion may be output. The electrocardiograms measured at different times may be divided for respective leads and then input into respective algorithms, the features or output values extracted through the algorithms may be combined at the rear ends, and then a final conclusion may be output.

In this case, when electrocardiograms at two or more times are mixed and used, the electrocardiograms may be input in an asynchronously state, or may be combined by being synchronized on a per beat basis or being synchronized based on deep learning and then used.

Furthermore, the integrated reading unit 140 may diagnose and predict diseases of the circulatory system, endocrine, nutritional and metabolic diseases, neoplastic diseases, mental and behavioral disorders, diseases of the nervous system, diseases of the eyes and their appendages, diseases of the ears and mastoids, diseases of the respiratory system, diseases of the digestive system, diseases of the skin and skin tissue, diseases of the musculoskeletal system and compressive tissue, diseases of the genitourinary system, pregnancy, childbirth and postpartum diseases, and congenital anomalies, deformities and chromosomal abnormalities.

In addition, through the integrated reading unit 140, the damage caused by physical trauma may be identified, the prognosis thereof may be checked and the pain thereof may be measured, the risk of death or worsening attributable to trauma may be predicted, concurrent complications may be detected or predicted, and specific conditions appearing before or after birth may also be identified.

Furthermore, through the integrated reading unit 140, for the healthcare field, the health condition of an examinee, which may lead to services such as aging, sleep, weight, blood pressure, blood sugar, oxygen saturation, metabolism, stress, tension, fear, drinking, smoking, problem behavior, lung capacity, exercise volume, pain management, obesity, body mass, body composition, diet, type of exercise, lifestyle pattern recommendations, emergency management, chronic disease management, medication prescription, test recommendation, checkup recommendation, nursing, telehealth management, telemedicine, vaccination, and post-vaccination management, may be measured, diagnosed, examined, and predicted.

Not only health conditions attributable to the individual diseases described above but also complex health conditions may be measured, diagnosed, examined and predicted, the worsening or alleviation of the health condition of an examinee may be predicted, short-term and long-term prognoses may be predicted, and the state of metastasis or complication from one disease to another may be predicted. The improvement or deterioration of health conditions attributable to specific medicines and the analysis and prediction of electrocardiograms may be trained. A specific medicine may be recommended according to a health condition.

Therefore, according to the configuration of the electrocardiogram reading system in which a deep learning-based model and a rule-based model are integrated, which has been described above, the misreading of electrocardiograms is prevented from being performed by the deep learning-based prediction unit by setting the readable range for final reading performed by the rule-based inference unit, the possibility of misreading and the accuracy of diagnosis are complemented by each other by enabling rapid and accurate reading to be performed by the deep learning-based prediction unit, the possibility of medical accidents is reduced by providing diagnostic information descriptive of the basis and reason for the diagnosis of disease, rapid medical response is enabled by rapidly performing final reading based on previous reading, and various healthcare services are enabled by applying the present invention not only to the medical field but also to the healthcare field utilizing various types of biometric data.

The embodiments described in the present specification and the configurations shown in the drawings are only the most preferred embodiments of the present invention and do not represent the overall technical spirit of the present invention. Accordingly, it should be understood that at the time when the present application is filed, there may be various equivalents and modifications that can replace the above embodiments and/or the configurations.

## Claims

1. An electrocardiogram reading system in which a deep learning-based model and a rule-based model are integrated, the electrocardiogram reading system comprising:
an electrocardiogram measurement unit configured to generate electrocardiogram data by measuring 1- or more-lead electrocardiograms;
a deep learning-based prediction unit configured to generate disease prediction information by diagnosing and predicting disease from the electrocardiogram data input from the electrocardiogram measurement unit through a deep learning algorithm constructed by being trained on training datasets of 1- or more-lead electrocardiograms and diseases corresponding to these electrocardiograms;
a rule-based inference unit configured to generate disease inference information by inferring disease from the electrocardiogram data input from the electrocardiogram measurement unit through a rule-based algorithm including a knowledge base, constructed with electrocardiogram data and disease information corresponding to the electrocardiogram data, and inference rules; and
an integrated reading unit configured to generate reading information by performing disease reading through an integrated analysis of the disease prediction information and the disease inference information and provide diagnostic information descriptive of a reason and basis for the diagnosis of the disease.

2. The electrocardiogram reading system of claim 1, wherein the rule-based inference unit sets a readable range for reading performed by the integrated reading unit.

3. The electrocardiogram reading system of claim 2, wherein, when the disease prediction information generated by the deep learning-based prediction unit is included in the readable range, the integrated reading unit generates and outputs the reading information and the diagnostic information, and, when the disease prediction information generated by the deep learning-based prediction unit is not included in the readable range, electrocardiograms are re-measured by the electrocardiogram measurement unit, or the disease prediction information is re-generated by readjusting parameters of the deep learning-based prediction unit.

4. The electrocardiogram reading system of claim 1, wherein the integrated reading unit performs final reading by using only the disease inference information generated by the rule-based inference unit.

5. The electrocardiogram reading system of claim 1, wherein a final diagnosis of the corresponding disease for the electrocardiogram data is made by the deep learning-based prediction unit and the rule-based inference unit.

6. The electrocardiogram reading system of claim 1, wherein:
primary reading information is generated by provisionally reading the electrocardiogram data through the deep learning-based prediction unit or the rule-based inference unit; and
the integrated reading unit comprises: a first reading unit configured to receive the primary reading information, and to allow a nurse, clinical pathologist, or emergency technician to log in, read the primary reading information, and generate secondary reading information; and a second reading unit configured to receive the secondary reading information, and to allow a cardiologist to log in, read the secondary reading information and generate final reading information.
